# EUROPEAN PATENT SPECIFICATION

(11) **EP 0 900 792 B1**
(45) Date of publication and mention of the grant of the patent: **29.10.2003**
(21) Application number: 98202832.6
(22) Date of filing: 24.08.1998
(51) Int. Cl.: C07D 263/58, C07D 209/34, A61K 31/42, A61K 31/38, A61K 31/47, C07D 209/08, C07D 265/36, C07D 215/227, C07D 279/16, C07D 401/12, C07D 409/12

(54) **Piperazine and piperidine derivatives as 5-HT1A and dopamine D2-receptor (ant)agonists**
Piperidin- und Piperazin Derivate als 5-HT1-Rezeptor-Agonisten
Dérivés de pipéridine et de pipérazine comme agonistes du récepteur 5-HT1

(30) Priority: 02.09.1997 EP 97202704
(43) Date of publication of application: 10.03.1999
(73) Proprietor: DUPHAR INTERNATIONAL RESEARCH B.V, 1381 CP Weesp (NL)
(72) Inventor: Feenstra, R.W., 1380 AC Weesp (NL); Visser, G.M., 1380 AC Weesp (NL); Kruse, C.G., 1380 AC Weesp (NL); Tulp, M.T.M., 1380 AC Weesp (NL); Long, S.K., 1380 AC Weesp (NL)
(74) Representative: Muis, Maarten

(56) References cited:
- EP-A- 0 169 148
- EP-A- 0 372 657
- EP-A- 0 512 755
- EP-A- 0 650 964
- EP-A- 0 785 195
- WO-A-94/06789
- WO-A-94/13659
- WO-A-94/15919
- WO-A-95/02592
- WO-A-96/03400
- WO-A-97/36893
- US-A- 5 486 518
- US-A- 5 519 025

## Description

The invention relates to a group of new piperazine compounds having interesting pharmacological properties. It has been found that compounds of the formula (a) 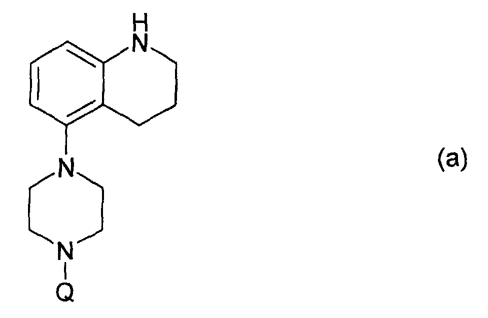 wherein:
Q is a group of the formula -CH₂-C(R₁R₂)-Z-R₃ wherein R₁ and R₂ represent H, C₁₋₄-alkyl or benzyl, Z is -C(R₄R₅)-C(=O)-, -C(R₄R₅)-O or -NH-C(=O)-, wherein R₄ and R₅ represent hydrogen or methyl, and R₃ is phenyl optionally substituted with halogen, CN, CH₃ or OCH₃, and salts thereof, have interesting pharmacological properties.

If Z is -NH-C(=O)- or -CH₂-O-, R₁=H and R₂ is alkyl or alkylphenyl, then the R-configuration at the chiral C-atom carrying R₁ and R₅, is preferred.

The serendipitous finding that buspirone, a non-benzodiazepine anxiolytic, appeared to be an agonist on 5-HT_{1A} receptors has triggered much research on ligands for this novel receptor, discovered by Gozlan et al. (Nature, 305, 140-142, 1983). Buspirone is a pyrimidinyl-piperazine derivative. Many patent applications describe phenyl-piperazine derivatives with high affinities for 5-HT_{1A} and other 5-HT₁ receptor subtypes, claimed to be useful as anxiolytics. Examples are WO 94/06789, WO 94/15919, WO 96/03400, US 5,486,518, US 5,519,025, EP 0169148, EP 0372657, EP 0512755. In none of these documents however, affinity for dopamine-D₂ receptors is mentioned. One exception is EP 0650964, also on phenyl-piperazines, but of the compounds of that invention it is specifically stated that they have a low affinity for dopamine-D₂ receptors. Also in applications on structurally related phenyl-piperidines with high affinity for 5-HT_{1A} receptors, e.g. WO 95/02592 and EP 0785195, affinity for dopamine-D₂ receptors is not mentioned.

An application in which compounds with a high affinity for 5-HT_{1A} as well as dopamine-D₂ receptors are mentioned is WO 94/13659. This invention relates to fused benzo compounds, including certain phenyl-piperazine derivatives. Although no specific examples were disclosed, some of these compounds are claimed to have both affinities. The only application which unambiguously claims a group of compounds which all show a high affinity for 5-HT_{1A} and dopamine-D₂ receptors is WO 97/36893, published after the priority date of the present invention.

It has now surprisingly been found that the compounds according to the invention show high affinity for both the dopamine D₂ and serotonin 5-HT_{1A} receptors (pKi range 7.0-9.5 for both receptor types). This combination is useful for the treatment of schizophrenia and other psychotic disorders and might allow for a more complete treatment of all disease symptoms: e.g. positive symptoms, negative symptoms and cognitive deficits.

The compounds show varying activities as either partial agonists or antagonists at dopamine D₂-, D₃- and D₄-receptors. Some compounds show agonist-like effects at dopamine receptors, however they potently antagonize apomorphine-induced climbing behaviour in mice (ED₅₀ values <1 mg/kg p.o). The compounds show varying activity as 5-HT_{1A} receptor agonists and induce aspects of the serotonin behavioural syndrome to differing intensities.

The compounds are active in therapeutic models sensitive to clinically relevant antipsychotics (e.g. the conditioned avoidance response; Van der Heyden & Bradford, Behav. Brain Res., 1988, 31:61-67), antidepressants (e.g. differential reinforcement of low rate responses; van Hest et al., Psychopharmacology, 1992, 107:474-479) and anxiolytics (e.g. suppresion of stress-induced vocalization; van der Poel et al., Psychopharmacology, 1989, 97: 147-148).

In contrast to clinically relevant dopamine D₂ receptor antagonists the described compounds have a low propensity to induce catalepsy in rodents and as such are likely to induce less extrapyramidal side effects than existing antipsychotic agents.

The 5-HT_{1A} receptor agonism inherent in these compounds may be responsible for the reduced tendency to induce extrapyramidal effects and the therapeutic effects observed in behavioural models sensitive to either antidepressants or anxiolytics.

The compounds are likely to be of value for the treatment of affections or diseases of the central nervous system caused by disturbances in either the dopaminergic or serotinergic systems, for example: aggression, anxiety disorders, autism, vertigo, depression, disturbances of cognition or memory and in particular schizophrenia and other psychotic disorders.

Suitable acids with which the compounds can form pharmaceutically acceptable acid addition salts are for example hydrochloric acid, sulphuric acid, phosphoric acid, nitric acid, and organic acids such as citric acid, fumaric acid, maleic acid, tartaric acid, acetic acid, succinic acid, benzoic acid, p-toluene sulphonic acid, methanesulphonic acid and naphtalene-sulphonic acid. The compounds of the invention can be brought into forms for administration by means of usual processes using auxiliary substances such as liquid and solid carrier materials.

The compounds of the invention can be obtained according to a number of synthetic routes (A to C) as described hereafter. The piperazine used is the compound with formula (b):

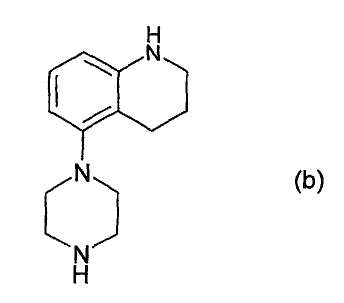

The synthesis of this piperazine has been described in EP 0189612. Its H-atom of the piperazine N-H moiety can be replaced by group Q in three different chemical ways (A, B and C, see below), eventually leading to the compounds of the invention.

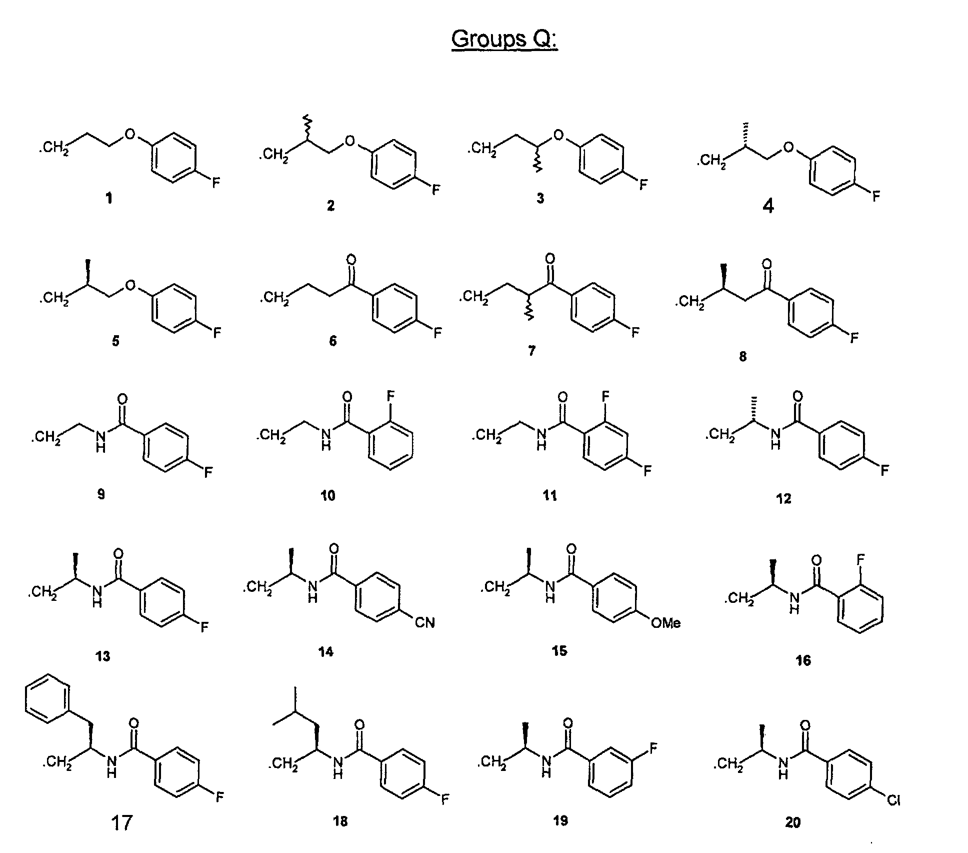

A number of Q-Cl or Q-Br compounds are commercially available, others can be obtained according to standard chemical procedures as illustrated in the examples showing the preparation of these intermediates.

### Synthesis routes A to C:

The compounds 1(A) - 6(A) listed in the table below were prepared via the synthesis depicted in scheme A (see below): piperazine (b) was reacted with **Q**-X (X = Cl, Br, OMs (Ms = mesyl), OTos (Tos = tosyl)) in *e*.*g*. acetonitrile with diisopropylethylamine (Et(*i*-Pr)₂N) acting as a base; in some cases KI (or Nal) was added. Triethylamine (Et₃N) can be used instead of Et(*i*-Pr)₂N.

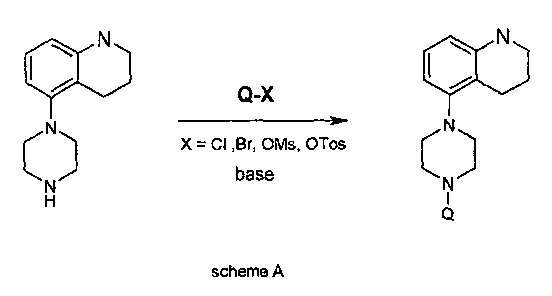

Compound 7(B) (see table below) was synthesized according to the reaction depicted in scheme B. Piperazine (b) was reacted with N-benzoyl-aziridines to yield the compounds of the invention.

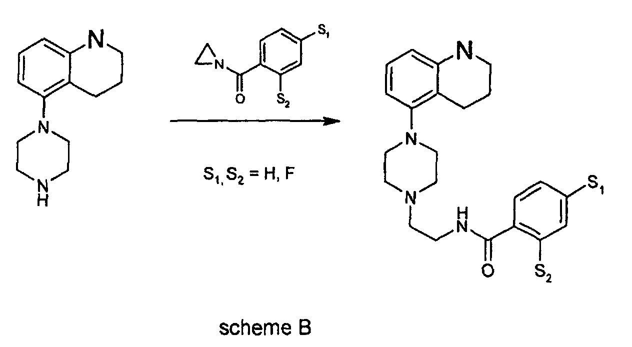

In scheme C, piperazine (b) is coupled to a protected amino acid (step 1) to yield an intermediate which, after two reductions (steps 2,3), yields an (optically active) primary amine. This amine can be derivatized easily by treatment with e.g. acid chlorides (step 4), yielding the compounds of the invention: see e.g. compounds 8(C)- 16(C) in the table. In this scheme, 1-hydroxybenzthiazole is abbreviated as HOBT.

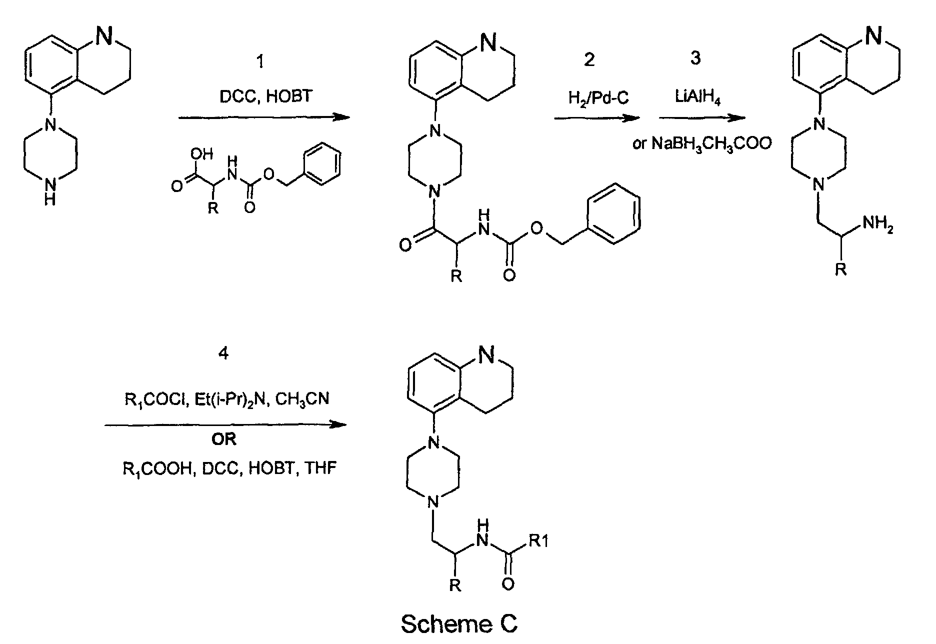

The preparation of the compounds of formula (a) and of a number of intermediate compounds will now be described in detail in the following examples.

### Examples:

### Example 1: preparation of compound 5(A).2HCl (see scheme A)

While stirring, 0.7 g (3.2 mmol) of piperazine (b) was dissolved in 20 ml of acetonitrile, after which 0.5 g (3.9 mmol) of diisopropylethylamine and 0.65 g (3.2 mmol) of Q6-CI were added. The reaction mixture was refluxed for 18 hrs after which the reaction was allowed to reach room temperature. The reaction mixture was concentrated *in vacuo*, after which the residue was subjected to column chromatography (SiO₂, eluent: CH₂Cl₂/MeOH = 98/2) yielding 0.4 g of an oil. The latter oil was treated with 2.5 equivalents of 0.5 M HCl/MeOH, yielding 0.4 g of almost pure compound 5(A).2HCI. m.p.: dec. > 260 °C. ¹H-NMR(CDCl₃/DMSO = 1/4) δ: 1.95(m, 2H), 2.13(m, 2H), 2.77(m, 2H), 3.10-3.30(cluster, 10H), 3.33(m, 2H), 3.40-3.90(cluster, 2H), 6.86-7.04(cluster, 2H), 7.24(t, 1H), 7.34(m, 2H), 8.08(m, 2H), 10.8(broad, 1H). In a similar way the examples 1(A) - 4(A) and 6(A) were prepared (see table below).

### Example 2: preparation of compound 7(B) (see scheme B)

A solution of 0.75 g (3.45 mmol) of piperazine (b) in 10 ml of dry tetrahydrofuran (THF) was added to a solution of 0.63 g (3.79 mmol) of N-(*para*-fluoro-benzoyl)aziridine in 8 ml of dry THF. The reaction mixture was stirred at reflux temperature for 2 hrs after which it was allowed to reach room temperature. The solvent was removed *in vacuo*, the residue subjected to flash column chromatography (SiO₂, eluent: CH₂Cl₂/MeOH = 98/2) yielding 0.51 g (39%) of glassy compound 7(B). m.p.: 150-2 °C. ¹H-NMR(CDCl₃/DMSO = 1/4) δ: 1.88 (m, 2H), 2.55-2.65(cluster, 8H), 2.94(m, 4H), 3.30(m, 2H), 3.57(m, 2H), 3.87(broad, 1H), 6.26(d, 1H, J=8Hz), 6.38(d, 1H, J=8Hz), 6.87(t broad, 1H), 6.95(t, 1H, J=8Hz), 7.13(m, 2H), 7.81(m, 2H).

### Example 3: preparation of compounds by routes outlined in scheme C

Step 1 (scheme C): 3.2 g (15 mmol) of a piperazine with formula: 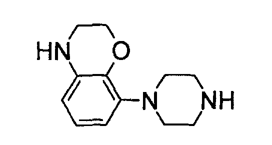 was dissolved in 45 ml of dry THF. While stirring and under a nitrogen atmosphere, 3.35 g (15 mmol) of (R)-N-(benzyloxycarbonyl)-alanine and 2.03 g (15 mmol) of 1-hydroxybenztriazol were added to the solution. The resulting mixture was brought to 0°C, after which 3.0 g (15 mmol) of dicyclohexylcarbodiimide (DCC) were added. After 1.5 hrs the precipitate was removed by filtration. To the filtrate SiO₂ was added after which the solvent was removed. The resulting dry SiO₂ powder with the reaction mixture absorbed on it, was placed on top of a dry SiO₂ column (flexible), after which elution was performed with ethyl acetate(EtOAc)/MeOH/NH₄OH = 97/2.7/0.3. The product-containing part of the column was cut out and the product was washed from the SiO₂ with MeOH. The resulting MeOH solution was concentrated and subsequently EtOAc (and a little absolute MeOH) was added, the latter solution being dried on MgSO₄. After removal of the drying agent and the solvent *in vacuo*, 6.1 g (96%) of the desired intermediate was isolated.
Step 2 (scheme C): 6.1 g (14 mmol) of the latter intermediate were dissolved in a mixture of 30 ml EtOAc and 70 ml of MeOH after which a catalytic amount of 10% Pd-C was added. Subsequently the mixture was hydrogenated at atmospheric pressure. After 16 hrs an extra amount of 10% Pd-C was added. Another 16 hrs later, the reaction mixture was filtered and concentrated *in vacuo*, leaving a residue which was subjected to flash column chromatography (SiO₂, eluent: CH₂Cl₂/MeOH/NH₄OH = 92/7.5/0.5), yielding 2.05 g (50%) of the primary amine.
Step 3 (scheme C): 2.05 g (7.1 mmol) of the latter primary amine were suspended in 9 ml of 1,2-dimethoxy-ethane, after which 1.32 g (35 mmol, 5 eq.) of NaBH₄ were added. Then the mixture was brought to 0 °C, after which a solution of 2.1 g (35 mmol) acetic acid in 7 ml 1,2-dimethoxy-ethane was carefully added to the mixture. When the addition was completed, the reaction mixture was brought to 85 °C. After a period of 1 hour the reaction was allowed to reach room temperature, the reaction mixture was acidified to pH 2 (aqueous 2 M HCI) and heated again until the temperature reached 60 °C, which situation was continued for 0.5 hrs. After this period, the reaction mixture was allowed to reach room temperature again and its pH was adjusted to 7-8 by adding aqueous 2 M NaCI. Extraction was performed with EtOAc, the organic fraction was washed with saturated NaCl/water and dried on Na₂SO₄. After removal of the drying agent and the solvent *in vacuo*, 0.73 g (37%) of a yellow oil containing the reduced amine intermediate was obtained. This was used without further purification for step 4 (see below).
Step 4 (scheme C): 0.73 g (2.6 mmol) of the reduced amine intermediate and 0.53 g (5.3 mmol) of Et₃N were dissolved in 35 ml CH₃CN. While stirring, a solution of 0.37 g (2.36 mmol) *para*-fluorobenzoylchloride in 7 ml of CH₃CN was added slowly and dropwise. After 16 hrs the reaction mixture was concentrated *in vacuo*, leaving a residue which was subjected to flash column chromatography (SiO₂, eluent: CH₂Cl₂/MeOH = 97/3), yielding an oil (free base) which was treated with 2.0 equivalents of 0.5 M HCl/MeOH to give 0.3 g (24%) of the hygroscopic di-HCI salt of the compound of the following structure: 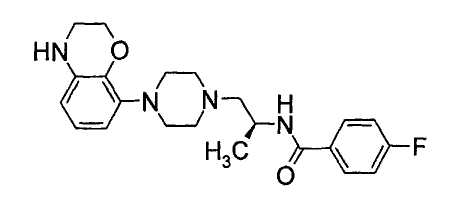 [α]_{D}²⁵ -59° (MeOH, 12.0 mg/ml). ¹H-NMR(CDCl₃/DMSO = 1/4) δ: 1.28(d, 3H, J=6 Hz), 3.0-3.7(cluster, 12H), 3.7-4.2(broad, 2H), 4.25(t, 2H, J=3 Hz), 4.57(m, 1H), 6.44-6.60(cluster, 2H), 6.72(t, 1H, J≈8 Hz), 7.25(m, 2H), 8.08(m, 2H), 8.8(d, 1H, J≈8 Hz), 10.3 (s broad, 1H). In a similar way the compounds 8(C) - 16(C) were prepared.

**Table**

| compound | Q | X | salt | melting point °C |
|---|---|---|---|---|
| | | | | |
| **1(A)** | 1 | Cl | 2HCl | 218-20 |
| **2(A)** | 2 | Cl | 2HCl | 190-2 |
| **3(A)** | 3 | OTos | 2HCl | 225-9 |
| **4(A)** | 5 | OMs | FUM | foam |
| **5(A)** | 6 | Cl | 2HCl | >260 d |
| **6(A)** | 7 | Br | 2HCl | >140 d |
| | | | | |
| **7(B)** | 9 | n.a. | base | 150-2 |
| | | | | |
| **8(C)** | 12 | n.a. | 2HCl | 238-43 |
| **9(C)** | 13 | n.a. | 2HCl | 240-3 |
| **10(C)** | 14 | n.a. | 2HCl | 193-9 d |
| **11(C)** | 15 | n.a. | 2HCl | 175-80 d |
| **12(C)** | 16 | n.a. | 2HCl | 173-8 d |
| **13(C)** | 17 | n.a. | 2HCl | 220-2 |
| **14(C)** | 18 | n.a. | 2HCl | 225 |
| **15(C)** | 19 | n.a. | 2HCl | >165 d |
| **16(C)** | 20 | n.a. | 2HCl | 178-83 d |
| *(A): compounds synthesized by routes outlined in Scheme A* | | | | |
| *(B): compounds synthesized by routes outlined in Scheme B* | | | | |
| *(C): compounds synthesized by routes outlined in Scheme C* | | | | |
| *d: decomposition* | | | | |
| *n.a.: not applicable* | | | | |

### Intermediates Q-Cl and Q-Br:

**Q2**-Cl was prepared from commercially available 3-bromo-2-methyl-1-chloro-propane and *para*-fluorphenol: 2.45 g (106.5 mmol) of Na was reacted with 75 ml of absolute EtOH after which, while stirring, 10.0 g (89 mmol) of *para*-fluorphenol was added. The resulting solution was added dropwise to a warm solution of 45.8 g (267 mmol, 3 eq.) of 3-bromo-2-methyl-1-chloro-propane in about 20 ml of absolute EtOH. Stirring at reflux temperature was continued for 40 hrs. After cooling, the precipitate was removed by filtration, subsequently the filtrate was concentrated *in vacuo* and the residue treated with 2 M NaOH. The resulting solution was extracted with Et₂O. The combined organic fractions were washed with water and dried on Na₂SO₄. After removal of the drying agent and the solvent *in vacuo*, 26 g of a yellow oil was left. Dry chromatography (SiO₂, eluent: petroleum benzin) yielded 9.9 g (54%) of **Q2**-Cl. This amount was contaminated (ca. 30%) with the HCI eliminated side product of **Q2**-CI. However, this batch was well suited for reaction with piperazine (b) (see scheme A).

**Q7**-Br was prepared in a Friedel-Craft reaction from *racemic* 4-bromo-2-methyl-butyryl bromide (for preparation see E.E. Ziegler et.al., *J. Am. Chem. Soc.,* **112**(1990)2755) and fluorobenzene: while stirring at room temperature and under a nitrogen atmosphere, 6.8 g (51 mmol) of AlCl₃ were suspended in 70 ml of 1,2-dichloro-ethane. Then 11.5 g (47 mmol) of *racemic* 4-bromo-2-methyl-butyryl bromide were added dropwise to the mixture. After 10 minutes the mixture was brought to 15 °C after which 14 ml (149 mmol, 3.2 eq.) of fluorobenzene was added dropwise. No change in temperature occurred. Stirring was continued for 18 hrs at room temperature, after which the reaction mixture was worked up carefully with water (temperature was kept below 40 °C). An extra amount of 1,2-dichloro-ethane was added. The biphasic system was separated and the organic layer was washed with water. Subsequently the organic layer was dried on Na₂SO₄. After removal of the drying agent and the solvent *in vacuo*, 10.7 g (88%) of **Q7**-Br resulted as a yellow oil. This almost pure oil was used for the reactions with piperazine (b) (see scheme A).

**Q8**-Br was prepared in a two-step synthesis; step 1: commercially available (S)-2-oxo-4-methyl-tetrahydrofuran was reacted with PBr₃ according to the procedure described by E.E. Ziegler et.al., *J. Am. Chem. Soc.,* **112**(1990)2755, giving (S)-4-bromo-3-methyl-butyryl bromide in 39% yield. Step 2: the latter compound was reacted with fluorobenzene in a Friedel-Craft reaction according to the procedure given for **Q7**-Br yielding 78% of the wanted product (se above). The obtained **Q8**-Br appeared not to be a good alkylating agent, therefor the diethylketal was prepared by treating **Q8**-Br with tri-ethoxymethane: 1.0 g (3.9 mmol) of **Q8**-Br was dissolved together with 1.14 g (7.72 mmol) of ethylorthoformiate (CH₃CH₂O)₃CH in 1.0 ml of absolute ethanol. After 5 minutes 1 small drop of 5 M H₂SO₄ was added. Stirring was continued for 16 hrs at room temperature after which the reaction mixture was concentrated *in vacuo*, leaving a dark residue of crude diethylketal of **Q8**-Br.

**Q3**-OH was prepared from 3-hydroxy-1-butanol in three steps according to the procedure described in EP89009149: the first step being the protection of the primary alcohol group with the *tert*. butylcarbonyl moiety, the second step being a Mitsunobu reaction between the latter compound and *para*-fluorphenol. The third step, the deprotection of the primary hydroxy group, gave the desired **Q3**-OH in 32% overall yield.

**Q4**-OH was prepared from commercially available (S)-3-bromo-2-methyl-1-propanol and *para*-fluorphenol: 7.56 g (49.4 mmol) of (S)-3-bromo-2-methyl-1-propanol and 11 g (98 mmol) of *para*-fluorphenol were dissolved in 150 ml of acetone after which 18 g (130 mmol) of powdered K₂CO₃ were added. The reaction mixture was brought to refluxing temperature for a period of 24 hrs. After the reaction mixture had reached roomtemperature, the solvent was removed *in vacuo* leaving a residu which was dissolved in ethyl acetate/water. The organic layer was washed subsequently with 2 N NaOH and water (2x), after which the solution was dried on MgSO₄. After subsequent removal of the drying agent and the solvent, the residu was subjected to flash chromatography (SiO₂, eluent: petroleum benzin/methyl-*tert*.-butyl ether 2/1) which eventually yielded 4.65 g (25.1 mmol, 51%) of a colorless oil consisting of **Q4**-OH.

**Q5**-OH was prepared from commercially available (R)-3-bromo-2-methyl-1-propanol and *para*-fluorphenol in the same way as **Q4**-OH (see above).

The above described **Q**-OH were converted into their corresponding mesylates and tosylates by standard procedures, e.g. MsCl/Et₃N and TosCl/pyridin respectively.

**Q9, Q10** and **Q11** are introduced in the compounds as described in scheme B (see above).

**Q12-Q20** are introduced in the compounds as described in scheme C (see above).

## Claims

1. Compounds having formula (a) 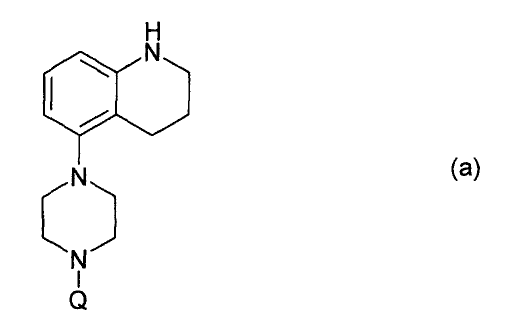 wherein
Q is a group of the formula -CH₂-C(R₁R₂)-Z-R₃ wherein R₁ and R₂ represent H, C₁₋₄-alkyl or benzyl, Z is -C(R₄R₅)-C(=O)-, -C(R₄R₅)-O or -NH-C(=O)-, wherein R₄ and R₅ represent hydrogen or methyl, and R₃ is phenyl optionally substituted with halogen, CN, CH₃ or OCH₃, and salts thereof.

2. Pharmaceutical compositions which contain at least one compound as claimed in claim 1 as an active component.

3. Method of preparing compositions for treating CNS-disorders, **characterized in that** a compound as claimed in claim 1 is brought into form suitable for administration to a patient.

4. Use of a compound as defined in claim 1 for the preparation of a composition for treating CNS-disorders, **characterised in that** a compound as claimed in claim 1 is used.

5. Use of a compound as defined in claim 1 for the preparation of a composition for treating schizohrenia, **characterised in that** a compound as claimed in claim 1 is used.

## Patentansprüche

1. Verbindungen mit der Formel (a) 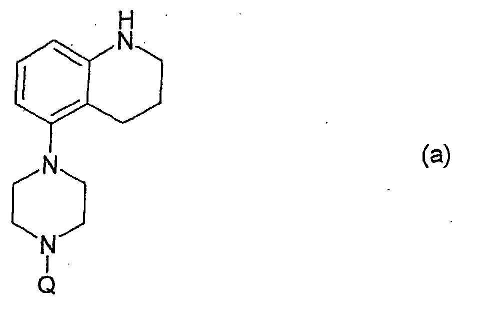 wobei
Q eine Gruppe der Formel -CH₂-C(R₁R₂)-Z-R₃ ist, wobei R₁ und R₂ H, ein C₁₋₄-Alkyl oder Benzyl darstellen, Z -C(R₄R₅)-C(=O)-, -C(R₄R₅)-O oder -NH-C(=O)- ist, wobei R₄ und R₅ Wasserstoff oder Methyl darstellen und R₃ ein Phenyl ist, das gegebenenfalls mit Halogen, CN, CH₃ oder OCH₃ und Salzen davon substituiert ist.

2. Pharmazeutische Zusammensetzungen, welche als Wirkstoff zumindest eine wie in Anspruch 1 beanspruchte Verbindung enthalten.

3. Verfahren zur Herstellung von Zusammensetzungen zur Behandlung von ZNS-Störungen, **dadurch gekennzeichnet, dass** eine wie in Anspruch 1 beanspruchte Verbindung in eine für die Verabreichung an einen Patienten geeignete Form gebracht wird.

4. Verwendung einer wie in Anspruch 1 definierten Verbindung zur Herstellung einer Zusammensetzung zur Behandlung von ZNS-Störungen, **dadurch gekennzeichnet, dass** eine wie in Anspruch 1 beanspruchte Verbindung verwendet wird.

5. Verwendung einer wie in Anspruch 1 definierten Verbindung zur Herstellung einer Zusammensetzung zur Behandlung von Schizophrenie, **dadurch gekennzeichnet, dass** eine wie in Anspruch 1 beanspruchte Verbindung verwendet wird.

## Revendications

1. Composés ayant la formule (a) : 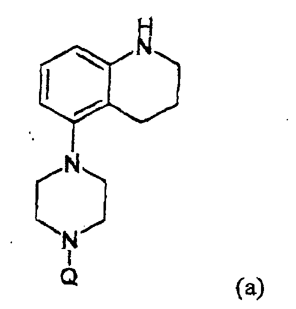 dans laquelle Q est un groupe de forme -CH₂-C(R₁R₂)-Z-R₃ où R₁ et R₂ représentent H, un groupe alkyle en C₁₋C₄ ou benzyle, Z est -C(R₄R₅)-C(=O)-, -C(R₄R₅)-O ou -NH-C(=O)-, où R₄ et R₅ représentent de l'hydrogène ou un groupe méthyle, et R₃ est un groupe phényle éventuellement substitué par un halogène, CN, CH₃ ou OCH₃ et leurs sels.

2. Compositions pharmaceutiques qui contiennent au moins un composé selon la revendication 1 comme composant actif.

3. Procédé de préparation de compositions pour le traitement de troubles du système nerveux central, **caractérisé en ce qu'**un composé selon la revendication 1 est présenté sous une forme convenant à une administration à un patient.

4. Utilisation d'un composé selon la revendication 1 pour la préparation d'une composition pour le traitement de troubles du système nerveux central, **caractérisée en ce qu'**on utilise un composé selon la revendication 1.

5. Utilisation d'un composé selon la revendication I pour la préparation d'une composition pour le traitement de la schizophrénie, **caractérisée en ce qu'**on utilise un composé selon la revendication 1.
